# EUROPEAN PATENT APPLICATION

(11) **EP 4 834 236 A1**
(43) Date of publication of application: **04.11.2026**
(21) Application number: 25173242.6
(22) Date of filing: 29.04.2025
(51) Int. Cl.: A61B 8/00, A61B 8/08

(54) **METHODS AND SYSTEMS FOR DETERMINING AN OPTIMAL DEPTH AND ZOOM SETTING FOR ULTRASOUND SCANS**

(71) Applicant: Koninklijke Philips N.V., 5656 AE Eindhoven (NL)
(72) Inventor: ANURADHA, Anuradha, Eindhoven (NL); SANTHANA NAIDU, Navaneetha Krishnan, Eindhoven (NL); V, Manikanda Krishnan, 5656AG Eindhoven (NL); VAJINEPALLI, Pallavi, Eindhoven (NL)
(74) Representative: Philips Intellectual Property & Standards

(57) **Abstract**

Apparatuses, systems, and methods for determining an optimal depth and zoom setting for ultrasound scans may cause an ultrasound probe to acquire a plurality of images acquired at a corresponding plurality of depths, analyze, using an artificial intelligence (AI) model, data corresponding to the plurality of images to estimate a depth setting and a zoom setting for performing ultrasound scanning, and cause the ultrasound probe to perform an ultrasound scan based on the depth setting and the zoom setting. In some instances, the system may analyze, using the AI model, data corresponding to the plurality of images to estimate a zoom setting for performing ultrasound scanning and cause the ultrasound probe to perform an ultrasound scan based on the zoom setting. In some implementations, the system may detect fetal anatomies using the AI model.

## Description

### FIELD OF THE INVENTION

The present disclosure relates to acquiring and analyzing medical imaging data. For example, embodiments herein relate to acquiring, and analyzing ultrasound imaging data using artificial intelligence (AI) model(s).

### BACKGROUD OF THE INVENTION

Ultrasound imaging is a widely used diagnostic tool for monitoring the health of a fetus. In rural and under resourced communities, the scarcity of ultrasound imaging results in a considerable gap in the healthcare for fetal care before, during, and after pregnancy. Additionally, rural health workers without prior ultrasound experience (e.g., midwives, nurses) may not trained on ultrasound scanning protocols or be able to properly adjust settings of ultrasound scanning procedures such as depth settings and zoom settings necessary to produce ultrasound images suitable for diagnostics. Accordingly, systems that allow less skilled users to obtain suitable ultrasound images are desired.

### SUMMARY OF THE INVENTION

Apparatuses, systems, and methods for determining an optimal depth and zoom setting for ultrasound scans may cause an ultrasound probe to acquire a plurality of images acquired at a corresponding plurality of depths, analyze, using an artificial intelligence (AI) model, data corresponding to the plurality of images to estimate a depth setting and a zoom setting for performing ultrasound scanning, and cause the ultrasound probe to perform an ultrasound scan based on the depth setting and the zoom setting. In some instances, the system may analyze, using the AI model, data corresponding to the plurality of images to estimate a zoom setting for performing ultrasound scanning and cause the ultrasound probe to perform an ultrasound scan based on the zoom setting. In some implementations, the system may detect fetal anatomies using the AI model.

The invention is defined by the independent claims. Advantageous embodiments are provided in the dependent claims.

In accordance with an aspect of the present invention, a computer-implemented method for determining an ultrasound depth setting and zoom setting is disclosed. The computer-implemented method comprises: causing an ultrasound probe to acquire a plurality of ultrasound images at a corresponding plurality of depths in a subject; analyzing the plurality of ultrasound images to determine a depth setting and a zoom setting for performing an ultrasound scan; and causing the ultrasound probe to perform the ultrasound scan based on the depth setting and the zoom setting.

In some embodiments, analyzing the plurality of ultrasound images to determine the depth setting and the zoom setting may comprise using an artificial intelligence (AI) model to estimate the depth setting and the zoom setting based on data corresponding to the plurality of ultrasound images.

In some embodiments, the method may further comprise determining whether a fetal anatomy is detected in an ultrasound image of the plurality of ultrasound images at a first depth of the plurality of depths. Preferably, the method may further comprise determining whether there is a first hyper echoic region beneath the fetal anatomy. More preferably, the method further comprises detecting a deepest pocket in the ultrasound image at the first depth and determining if the deepest pocket comprises the first hyper echoic region.

In such embodiments, the method may further comprise determining whether the first hyper echoic region meets a threshold size. Preferably, the method may further comprise determining the zoom setting based on the threshold size.

In some embodiments, the method further comprises determining whether the first hyper echoic region comprises a placenta.

In some embodiments, the method may further comprise determining whether there is a second hyper echoic region beneath the first hyper echoic region.

In some embodiments, the method may further comprise detecting a fetal anatomy based on an anatomy detector model. Preferably, the method may further comprise applying a bounding box to the fetal anatomy.

In some embodiments, the method may further comprise, in response to determining that no fetal anatomy is detected at the first depth, determining whether the fetal anatomy is detected in an ultrasound image of the plurality of ultrasound images at a second depth of the plurality of depths.

In accordance with another aspect of the present invention, a non-transitory computer-readable medium is disclosed. The non-transitory computer-readable medium carries instructions that, when executed by at least one processor of an ultrasound imaging system comprising an ultrasound probe configured to acquire ultrasound images, cause the ultrasound imaging system to perform the computer-implemented method of any of the embodiments described above.

In accordance with yet another aspect of the present invention, an ultrasound imaging system is disclosed. The ultrasound imaging system comprises an ultrasound probe configured to acquire ultrasound images, and at least one processor configured to carry out the steps of the computer-implemented method of any of the embodiments described above.

In accordance with at least one example disclosed herein, an ultrasound imaging system is disclosed. The ultrasound imaging system comprises an ultrasound probe configured to acquire ultrasound images and at least one processor configured to cause the ultrasound probe to acquire a plurality of ultrasound images at a plurality of depths in a subject. The at least one processor is further configured to analyze the plurality of ultrasound images to determine a depth setting for performing an ultrasound scan. The at least one processor is further configured to cause the ultrasound probe to perform the ultrasound scan with the depth setting.

In some embodiments, the at least one processor is further configured to determine whether a fetal anatomy is detected in an ultrasound image of the plurality of ultrasound images at a first depth of the plurality of depths. In some such embodiments, the at least one processor is further configured to detect a deepest pocket in the ultrasound image at the first depth and determine if the deepest pocket comprises a first hyper echoic region underneath the fetal anatomy. In some such embodiments, the at least one processor is further configured to determine whether the first hyper echoic region comprises a placenta. In some such embodiments, the at least one processor is further configured to determine whether there is a second hyper echoic region beneath the first hyper echoic region. In some such embodiments, the at least one processor is further configured to determine that the first hyper echoic region meets a threshold size.

In some embodiments, the at least one processor is further configured to detect a fetal anatomy based on an anatomy detector model. In some such embodiments, the at least one processor is further configured to apply a bounding box to the fetal anatomy.

In some embodiments, the at least one processor is further configured to determine a zoom setting for performing the ultrasound scan.

In accordance with at least one example disclosed herein, at least one non-transitory computer-readable medium carrying instructions is disclosed. When executed by at least one processor of an ultrasound imaging system, the instructions cause the ultrasound imaging system to perform an ultrasound depth scan corresponding to a depth estimation mode to obtain ultrasound imaging per depth. The instructions, when executed by the at least one processor, further cause the ultrasound imaging system to analyze, using an artificial intelligence (AI) model, data corresponding to the ultrasound imaging per depth to estimate a depth setting for performing ultrasound scanning. The instructions, when executed by the at least one processor, further cause the ultrasound imaging system to perform an ultrasound scan based on the depth setting.

In some embodiments, the instructions, when executed by the ultrasound imaging system, further cause the ultrasound imaging system to determine whether a fetal anatomy is detected in the ultrasound depth scan at a first depth. In some such embodiments, the instructions, when executed by the ultrasound imaging system, further cause the ultrasound imaging system to determine whether a deepest pocket of the ultrasound imaging at the first depth comprises a first hyper echoic region underneath the fetal anatomy. In some such embodiments, the instructions, when executed by the ultrasound imaging system, further cause the ultrasound imaging system to determine whether there is a second hyper echoic region beneath the first hyper echoic region.

In some embodiments, the instructions, when executed by the ultrasound imaging system, further cause the ultrasound imaging system to determine that the first hyper echoic region meets a threshold size.

### BRIEF DESCRIPTION OF THE DRAWINGS

Fig. 1 illustrates a block diagram of an ultrasound imaging system arranged in accordance with principles of the present disclosure.
Fig. 2 illustrates an example of a high level optimal depth and zoom estimation algorithm for optimal depth and zoom estimation, according to at least one embodiment of the present disclosure.
Fig. 3 illustrates an example optimal depth selection AI algorithm, according to at least one embodiment of the present disclosure.
Fig. 4 illustrates an example of an optimal zoom selection AI algorithm used to estimate an optimal zoom setting, according to at least one embodiment of the present disclosure.
Fig. 5 illustrates an example of a uterine wall as a posterior extent, according to at least one embodiment of the present disclosure.
Fig. 6 illustrates an example of a bounding box limited by a thin posterior uterine wall, according to at least one embodiment of the present disclosure.
Fig. 7 illustrates an example of a placenta as a posterior anatomy of the uterus, according to at least one embodiment of the present disclosure.
Fig. 8 illustrates a method for determining an ultrasound depth setting and zoom setting, according to at least one embodiment of the present disclosure.

### DETAILED DESCRIPTION OF EMBODIMENTS

The following description of certain examples is illustrative in nature and is in no way intended to limit the disclosed technology or its applications or uses. In the following detailed description of examples of the present apparatuses, systems, and methods, reference is made to the accompanying drawings, which form a part hereof, and in which are shown by way of illustration specific examples in which the described apparatuses, systems, and methods may be practiced. These examples are described in sufficient detail to enable those skilled in the art to practice the presently disclosed apparatuses, systems, and methods, and it is to be understood that other examples may be utilized and that structural and logical changes can be made without departing from the spirit and scope of the present disclosure. Moreover, for the purpose of clarity, detailed descriptions of certain features will not be discussed when they would be apparent to those with skill in the art, so as not to obscure the description of the present technology. The following detailed description is therefore not to be taken in a limiting sense, and the scope of the present technology is defined only by the appended claims.

In rural areas, midwives, nurses, or other healthcare workers may use portable ultrasound systems (e.g., tablet-based) where they make sweeps with an ultrasound probe across the abdomen in a pre-defined pattern across the mother's abdomen to acquire fetal images. The healthcare workers may not see the acquired images during the scan, so it may be referred to as a "blind sweep." The acquired images are analyzed later by a clinician such as an obstetrician or radiologist. The portable imaging system may have several default settings to make it easier for healthcare workers to acquire images with little ultrasound training. In some cases, particularly in B-mode imaging, the portable ultrasound imaging system may provide default depth settings based on a scan type (e.g., fetal scan, liver scan). Of course, this is merely one example, and other types of ultrasound imaging systems may have default/preset settings to assist novice users and/or save time for more skilled users.

However, in some cases the depth settings may need manual adjustment by skilled professionals due to subject-specific factors. For example in fetal scans, the gestational age (GA), maternal habitus, thick maternal abdominal wall, high body mass index (BMI) of the patient, polyhydramnios, oligo hydramnios, small for gestational age and large for gestational age babies, wrong last menstrual period (LMP), and intrauterine growth restriction (IUGR) may affect the ideal depth setting for imaging. It may be difficult for health care workers with little ultrasound training to perform such adjustments in various cases according to different patient anatomy to the degree of accuracy needed to produce useful/meaningful ultrasound imaging for analysis. As used herein, useful or meaningful images means images from which a radiologist or obstetrician can make a clinical measurement or diagnosis. For example, the wrong depth setting may lead to failure to capture desired anatomical views, unbalanced visualizations, and/or incomplete field of view of organ(s) of interest. In current practice, there is no way to automatically determine an optimal depth setting per patient to perform ultrasound scans at as to produce meaningful ultrasound imaging for analysis.

Further, ultrasound images obtained using non-optimal depth settings may produce poor quality images (e.g., artifacts, low signal to noise ratio, low resolution, objects of interest out of focus). In some cases, the poor-quality images acquired during the ultrasound scan using non-optimal depth settings may not be discovered until the images and data are reviewed later by a clinician. In the example above of healthcare workers visiting rural patients, the clinician would have to contact the rural healthcare worker, and the rural healthcare worker would have to revisit the patient to perform another ultrasound scan at a different depth setting. This delays care and increases the time the rural healthcare worker and patient needs to devote to scans. In remote areas or where rural healthcare workers have large caseloads, making additional appointments for scans may be difficult. Accordingly, automatically determining an optimal depth settings for ultrasound scanning during the initial ultrasound scan may be desirable. Such automatic determining of optimal depth settings may benefit skilled ultrasound users as well as it may save time during exams.

Users of medical imaging systems, such as ultrasound imaging systems, face technical challenges related to acquiring images using standard clinical protocols. For example, it may take two or more years to train sonographers to operate ultrasound imaging systems and adjust depth/zoom settings while operating the ultrasound imaging systems. Expert well-trained sonographers may be able to recognize certain aspects of the patient and anatomies in the imaging to adjust the depth settings in order to obtain useful images, but the same may not be said for novice users. Advancements in artificial intelligence (AI) and image processing may be used to support novice users (e.g., sonographers or clinicians) with less familiarity with adjusting depth settings. The AI and image processing may allow the automation and enhancement of diagnostic workflows. As a result, the novice user may be able to acquire useful imaging.

Embodiments herein introduce a method for using AI to measure the highest possible distance of the posterior most structure of the maternal uterus or the anatomy in question and calculate a best depth setting (optimal depth setting) used for performing the ultrasound scan. By leveraging AI models, embodiments herein may reduce or remove the need for manually adjusting the depth setting per individual patient. This automation may reduce the reliance on manual skills and knowledge (or lack thereof), which may allow novice health care workers to perform obstetric ultrasound scanning more efficiently and/or accurately. This may help save time for healthcare professionals and/or improve workflow efficiency. Further, the AI based method to determine optimal depth settings for performing ultrasound scans may be integrated into various ultrasound systems or imaging platforms, which may enhance capabilities and/or provide advanced diagnostic tools to novice healthcare providers.

It should be understood that an accurate depth setting is important for useful ultrasound image acquisition. Using an incorrect depth setting may hamper the diagnosis of many clinical conditions by not including correct anatomies in question and thus producing non-useful ultrasound imaging.

In some instances, routine and targeted obstetric ultrasound examinations may be a part of obstetric/radiology work which includes a depth setting determination to be completed at the beginning of the exam. However, while this may be easy for a skilled user (e.g., radiologist or a sonologist) to adjust the depth settings based on the individual patient GA and habitus, a novice user (e.g., health care worker, sonographer, or resident) may not be able to adjust the depth considering each patient's GA, habitus, fetal conditions like polyhydramnios or oligohydramnios. As a result, it may be beneficial, according to embodiments herein, to estimate an optimal depth setting for each patient using an AI model.

Additionally, traditional methods rely on manual depth adjustments by skilled users to set the depth suitable for each patient based on the GA, habitus, and fetal conditions as described above. This process can be skill based and prone to errors leading to potential diagnostic inconsistencies or inability to make a diagnosis at all (e.g., wrong views, incomplete field of view of desired anatomy). Further, existing ultrasound systems may offer basic depth setting presets (pre-configured settings) but do not estimate an optimal depth setting for each patient. This limitation can impact diagnostic accuracy, especially in cases with fetal conditions affecting the size of the uterus. Variations in individual habitus, maternal conditions different GA can affect the consistency and reliability of standard depth setting offered by the machine settings. In some embodiments, depth setting estimation may improve the workflow efficiency and patient throughput, particularly in busy clinical settings. Embodiments herein not only overcome the limitations of standard settings offered by one size fits all machine settings but also enables efficient customization of the depth settings to cater individual patient.

In some embodiments, before starting an ultrasound scan of a patient, the user (e.g., healthcare provider) enters the ultrasound system into an optimal depth estimation mode, for example, by clicking an on-screen option, or a button on the ultrasound probe. To start the optimal depth estimation scan, the user places the ultrasound probe at the highest point of the maternal belly perpendicular to the maternal spine. In some cases, this may be at or near the belly button of the patient. The user initiates the depth analysis, for example, by clicking an on-screen option. The system internally invokes an optimal depth estimator, which selects the optimal depth setting for the patient. The user may accept the estimated optimal depth setting and proceed with the ultrasound scan using the optimal depth setting provided by the optimal depth estimator.

In some implementations, an optimal zoom setting may also be estimated by the system and the user may accept the optimal zoom setting and proceed with the ultrasound scan based on the optimal depth setting and/or the optimal zoom setting. The zoom setting may be used to acquire an image of a region of interest within a field of view. For example, the zoom setting may be used to acquire an image of a fetal heart detected within an image of a fetus.

In some instances, in the optimal depth estimation mode, the ultrasound system acquires images at all available depths and stores one or more image(s) at each depth. For example, the system may have the capability to scan from an initial depth level D₁ to Dₙ where D₁ is the nearest to the probe/shallowest depth level and Dₙ is the furthest from the probe/deepest depth level. The system may set the depth at D₁ initially and acquire/store one or more image(s) at D₁. The system may set the depth at D₂ and acquire/store one or more image(s) at D₂ and so on for D₃, D₄, D₅, all the way to the furthest/deepest depth Dₙ. The stored images at each depth are provided to an optimal depth selection AI algorithm which estimates the optimal depth, Dₒ, based on the one or more images at each depth.

When a closer visualization of an organ or other anatomical feature would be beneficial, in some examples, an optimal zoom selection AI algorithm (used to estimate an optimal zoom setting) may select the starting depth Dₛ and final depth Dᵣ and the spatial span starting position Xₛ and spatial span final position X_{f} to be captured. Note that the spatial span positions Xₛ and X_{f} may be understood as side-to-side dimensions used for the zoom setting. The optima zoom selection AI algorithm may be invoked with the optimal depth selection AI algorithm when using the optimal depth estimator and/or may be used separately after the optimal depth has been determined.

The optimal depth estimator may analyze each image acquired at each depth using the optimal depth selection AI algorithm (and/or the optimal zoom selection AI algorithm. For example, the optimal depth selection AI algorithm may analyze images I₁, I₂, I₃, I₄,... I_{N} at depth D₁, and analyze I₁, I₂, I₃, I₄,... I_{N} at depth D₂,..., until the deepest depth (Dₙ) of the ultrasound scan is reached. The analysis may continue until an optimal depth setting is estimated or until all images at all depths are analyzed and no optimal depth setting is estimated. If the back uterine wall is detected and no other anatomy was detected between the ultrasound probe and the back uterine wall (or between the front and back uterine walls), in some cases, no optimal depth may be determined., In such case, the system may prompt the user to adjust the position of the ultrasound probe and perform the depth estimation scan again.

In some embodiments, each image at each depth may be analyzed using an optimal depth selection AI algorithm. The optimal depth selection AI algorithm may begin by passing an image (I₁ at depth D₁) through an anatomy detector. If an anatomy is detected (e.g., fetal parts or amniotic fluid), the algorithm determines if a deepest pocket of the image has a hyper echoic region beneath it. If an anatomy is not detected, the next image at the same depth is analyzed (I₂ at depth D₁). If a deepest pocket does have a hyper echoic region beneath it the algorithm may determine whether the hyper echoic region is a placenta or not. If the deepest pocket does not have a hyper echoic region beneath it, the next image (I₂) at the same depth (D₁) is analyzed.

If the hyper echoic region is a placenta the algorithm may determine if there is another (second) hyper echoic region beneath the placenta. If so, the algorithm may determine if the second hyper echoic region extends for a certain distance (meets a size threshold) (e.g., 2 cm). If the hyper echoic region extends for the certain distance, the current depth is the optimal depth setting.

If the hyper echoic region is determined to not be a placenta the algorithm may determine if the hyper echoic region that is the placenta extends for a certain distance (meets a size threshold) (e.g., 2 cm). If the hyper echoic region extends for the certain distance, the current depth is the optimal depth setting.

In some embodiments, an optimal zoom selection AI algorithm may be used to estimate an optimal zoom setting. For example, the optimal zoom selection AI algorithm may begin with analyzing images acquired at some or all depths. The image may be passed through an anatomy detector of the algorithm and the algorithm may determine whether an anatomy is detected. If yes an anatomy is detected, the algorithm may determine whether there is a hyper echoic region beneath the detected anatomy. If no anatomy is detected or if no hyper echoic region is found beneath a detected anatomy a next image at the same depth (or next depth) may be analyzed. If the algorithm determines that there is a hyper echoic region beneath the detected anatomy, the algorithm may determine if the hyper echoic region extends for a certain distance (meets a size threshold) (e.g., 2 cm). If so, the algorithm outputs an optimal zoom setting where the optimal zoom setting is an initial zoom setting of the detected anatomy (e.g., a bounding box) with the certain distance added and subtracted from the initial zoom setting. For example, if an initial zoom setting is Dₛ, D_{f}, Xₛ, X_{f}, where Dₛ is the starting depth, D_{f} is the final depth, X₋ₛ is the spatial span starting position and Xᵣ is the spatial span final position to be captured. and the certain distance is Z the estimated optimal zoom setting is Dₛ - Z, D_{f} + Z, Xₛ -Z and X_{f} - Z.

In some instances, to improve the confidence of the optimal zoom setting, optimal zoom settings may be found of other anatomies known to be positionally close the intended anatomy. For example, if the intended anatomy is a heart, an optimal zoom setting may be found for the heart-side ribs and the spine and compared to the optimal zoom setting of the heart to improve the confidence in the optimal zoom setting of the heart.

In some embodiments, the optimal depth estimation mode may be used to detect certain anatomies of the fetus. For example, the optimal zoom AI algorithm may detect a heart of the fetus and determine a bounding box of the detected heart.

Once estimated, the optimal depth setting and/or the optimal zoom setting may be displayed to the user for approval to be used, or may be automatically used by the ultrasound system for ultrasound scanning of the patient. In some cases, the optimal depth setting and/or the optimal zoom setting may be stored in memory of the system and used to further train the AI model/algorithms discussed herein or added to the patient's health data so that the optimal depth setting and/or optimal zoom setting may be reused in future ultrasound scanning. In some instances, the user may decline the optimal depth setting and/or the optimal zoom setting.

It should be understood that the optimal depth setting and optimal zoom setting estimation discussed herein refers to implementations using various ultrasound scanning procedures for fetal scans. However, in some implementations, the optimal depth setting and optimal zoom setting estimation may be used for estimating an optimal depth setting and optimal zoom setting for other nonfetal scanning procedures such as scanning procedures used for toddlers/teens/adults for the recognition of any individual anatomy concerned (e.g., abdomens or soft tissue scans).

The AI model(s) disclosed herein (e.g., optimal depth selection AI algorithm) may be trained using training datasets comprising ultrasound data acquired by ultrasound procedures, blind sweeps and/or other techniques. The training datasets may further include labels, annotations, or other data. For example, the training dataset for the AI model may include images of various patients at various depths and an optimal depth setting and optimal zoom setting for each patient. Once the AI model has been trained, the AI model receives data comprising ultrasound data, and AI model may estimate the optimal depth setting and/or optimal zoom setting.

The AI model may be a neural network in some embodiments. The neural network may include one or more input nodes that receive training datasets. The input nodes can correspond to functions that receive the input and produce results. These results can be provided to one or more levels of intermediate nodes that each produce further results based on a combination of lower-level node results. A weighting factor can be applied to the output of each node before the result is passed to the next layer node. At a final layer, ("the output layer,") one or more nodes can produce a value classifying the input that, once the model is trained, can be used to evaluate new data (e.g., estimated optimal depth/zoom settings). In some implementations, such neural networks, known as deep neural networks, can have multiple layers of intermediate nodes with different configurations, can be a combination of networks or machine learning/AI models that receive different parts of the input and/or input from other parts of the deep neural network, or are convolutions-partially using output from previous iterations of applying the neural network as further input to produce results for the current input. However, in other examples, other AI models may be used.

The AI model can be trained with supervised or unsupervised learning. For example, in supervised learning, the optimal depth detection portion of an AI model may be provided with a set of images with detected anatomy and an indication of the optimal depth. In some embodiments, the anatomy detector of the AI model may be trained with a set of images with included anatomy labeled. In some embodiments, the anatomy detector may be trained separately from the optimal depth detection algorithm. In some embodiments, they may be trained simultaneously. Testing data can then be provided to the AI model to assess accuracy. Testing data can be, for example, a portion of the entire dataset (e.g., 10%) held back to use for evaluation of the model. The output from the AI model can be compared to the desired or expected output for the training data and, based on the comparison, the AI model can be modified, such as by changing weights between nodes of the neural network and/or parameters of the functions used at each node in the neural network (e.g., applying a loss function). Based on the results of the AI model evaluation, and after applying the described modifications, the AI model can then be retrained to evaluate new data.

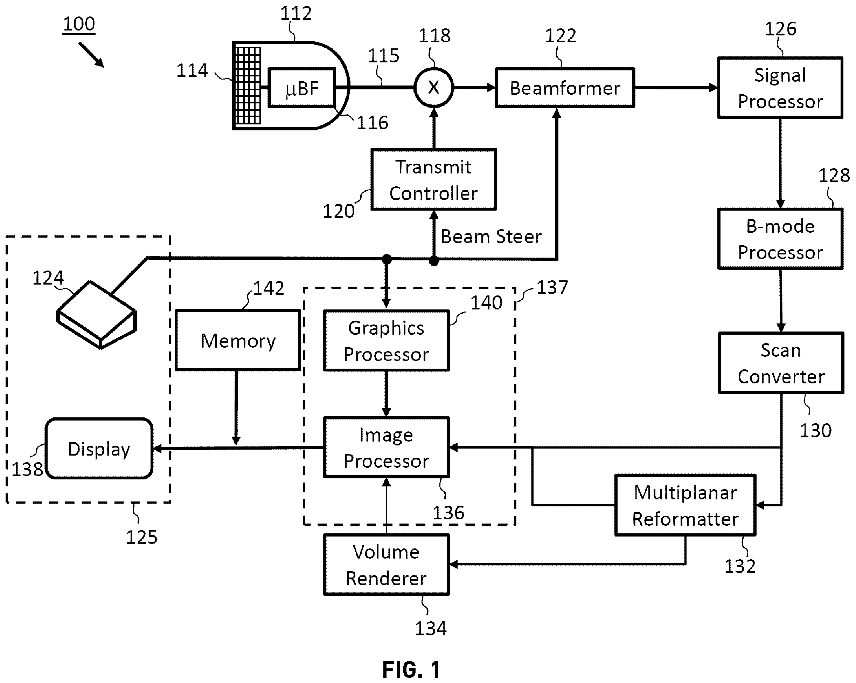

Fig. 1 illustrates a block diagram of an ultrasound imaging system 100 arranged in accordance with principles of the present disclosure. In the ultrasound imaging system 100 of Fig. 1, an ultrasound probe 112 includes a transducer array 114 for transmitting ultrasonic waves and receiving echo information. The transducer array 114 can be implemented as a linear array, convex array, a phased array, and/or a combination thereof. The transducer array 114, for example, can include a two-dimensional array (as shown) of transducer elements capable of scanning in both elevation and azimuth dimensions for 2D and/or 3D imaging. The transducer array 114 can be coupled to a microbeamformer 116 in the probe 112, which controls transmission and reception of signals by the transducer elements in the array. In this example, the microbeamformer 116 is coupled by the probe cable to a transmit/receive (T/R) switch 118, which switches between transmission and reception and protects the main beamformer 122 from highenergy transmit signals. In some embodiments, the T/R switch 118 and other elements in the system can be included in the ultrasound probe 112 rather than in a separate ultrasound system base. In some embodiments, the ultrasound probe 112 may be coupled to the ultrasound imaging system via a wireless connection (e.g., WiFi, Bluetooth).

The transmission of ultrasonic beams from the transducer array 114 under control of the microbeamformer 116 is directed by the transmit controller 120 coupled to the T/R switch 118 and the beamformer 122, which receives input from the user's operation of the user interface (e.g., control panel, touch screen, console) 125. The user interface 125 may include soft and/or hard controls. One of the functions controlled by the transmit controller 120 is the direction in which beams are steered. Beams may be steered straight ahead from (orthogonal to) the transducer array 114, or at different angles for a wider field of view. The partially beamformed signals produced by the microbeamformer 116 are coupled via channels 115 to a main beamformer 122 where partially beamformed signals from individual patches of transducer elements are combined into a fully beamformed signal. In some embodiments, microbeamformer 116 is omitted and the transducer array 114 is coupled via channels 115 to the beamformer 122. In some embodiments, the system 100 can be configured (e.g., include a sufficient number of channels 115 and have a transmit/receive controller programmed to drive the transducer array 114) to acquire ultrasound data responsive to a plane wave or diverging beams of ultrasound transmitted toward the subject. In some embodiments, the number of channels 115 from the ultrasound probe may be less than the number of transducer elements of the transducer array 114 and the system can be operable to acquire ultrasound data packaged into a smaller number of channels than the number of transducer elements.

The beamformed signals are coupled to a signal processor 126. The signal processor 126 can process the received echo signals in various ways, such as bandpass filtering, decimation, I and Q component separation, and/or harmonic signal separation. The signal processor 126 can also perform additional signal enhancement such as speckle reduction, signal compounding, and noise elimination. The processed signals are coupled to a B-mode processor 128, which can employ amplitude detection for the imaging of structures in the body. The signals produced by the B-mode processor 128 are coupled to a scan converter 130 and a multiplanar reformatter 132. The scan converter 130 arranges the echo signals in the spatial relationship from which they were received in a desired image format. For instance, the scan converter 130 can arrange the echo signal into a two-dimensional (2D) sector-shaped format, or a pyramidal three-dimensional (3D) image. The multiplanar reformatter 132 can convert echoes, which are received from points in a common plane in a volumetric region of the body into an ultrasonic image of that plane, as described in U.S. Pat. No. 6,443,896 (Detmer).

A volume renderer 134 converts the echo signals of a 3D data set into a projected 3D image as viewed from a given reference point, e.g., as described in U.S. Pat. No. 6,530,885 (Entrekin et al.). In some implementations, the system 100 can additionally or alternatively be configured to perform 3D and/or 4D acquisitions, such as protocolized 3D/4D acquisitions. The 2D or 3D images can be coupled from the scan converter 130, multiplanar reformatter 132, and volume renderer 134 to at least one processor 137 for further image processing operations. For example, the at least one processor 137 can include an image processor 136 configured to perform further enhancement and/or buffering and temporary storage of imaging data for display on an image display 138. However, in some embodiments, the display 138 may not display ultrasound images to the user.

The display 138 can include a display device implemented using a variety of display technologies, such as LCD, LED, OLED, or plasma display technology. The at least one processor 137 can include a graphics processor 140, which can generate graphic overlays for display with the ultrasound images. These graphic overlays can contain, e.g., standard identifying information such as patient name, date and time of the image, imaging parameters, and the like. For these purposes, the graphics processor 140 receives input from the user interface 125, such as a typed patient name. The user interface 125 can also be coupled to the multiplanar reformatter 132 for selection and control of a display of multiple multiplanar reformatted (MPR) images.

The user interface 125 can include one or more mechanical controls, such as buttons, dials, a trackball, a physical keyboard, and others, which may also be referred to herein as hard controls. Alternatively or additionally, the user interface 125 can include one or more soft controls, such as buttons, menus, soft keyboard, and other user interface control elements implemented for example using touchsensitive technology (e.g., resistive, capacitive, or optical touch screens). One or more of the user controls can be co-located on a control panel 124. For example, one or more of the mechanical controls can be provided on a console and/or one or more soft controls can be co-located on a touch screen, which can be attached to or integral with the console. The display 138 and the user interface 125 can be included in an I/O component, via which outputs are provided by the system 100 and/or inputs are received by the system 100. In some implementations, the user interface 125 can receive inputs and provide outputs of the disclosed system. For example, the user interface 125 can receive a user input specifying a type of exam, patient information, an acceptance, or a rejection of an optimal depth and/or zoom setting. In some implementations the one or more outputs of the system can be provided via one or more graphical user interfaces (e.g., via the display 138).

The at least one processor 137 (e.g., the image processor 136, the graphics processor 140, or a different processor) can perform functions associated with acquiring and configuring medical imaging data, as described herein. For example, the at least one processor 137 can identify imaging protocols, configure sweep direction based on user characteristics, receive acquired imaging data, configure the acquired imaging data based on an imaging protocol and the configured sweep direction, evaluate IMU data, and so forth.

Although described as separate processors, it will be understood that the functionality of any of the processors described herein can be implemented in a single processor (e.g., a CPU or GPU implementing the functionality of processor 137) or fewer number of processors than described in this example. In some embodiments, the at least one processor 137 can be hardware-based (e.g., include multiple layers of interconnected nodes implemented in hardware). In some embodiments, the at least one processor 137 can be implemented at other processing stages, e.g., prior to the processing performed by the image processor 136, volume renderer 134, multiplanar reformatter 132, and/or scan converter 130. In some embodiments, the at least one processor 137 can be implemented to process ultrasound data in the channel domain, beamspace domain (e.g., before or after beamformer 122), the IQ domain (e.g., before, after, or in conjunction with signal processor 126), and/or the k-space domain. As described, in some embodiments, functionality of two or more of the processing components (e.g., beamformer 122, signal processor 126, B-mode processor 128, scan converter 130, multiplanar reformatter 132, volume renderer 134, at least one processor 137, image processor 136, graphics processor 140, etc.) can be combined into a single processing unit and/or divided between multiple processing units. The processing units can be implemented in software, hardware, or a combination thereof. For example, the at least one processor 137 can include one or more graphical processing units (GPU). In another example, beamformer 122 can include an application specific integrated circuit (ASIC).

The at least one processor 137 can be coupled to one or more computer-readable media (e.g., memory 142) included in the system 100, which can be non-transitory. The one or more computer-readable media can carry instructions and/or a computer program that, when executed, cause the at least one processor 137 to perform operations described herein. A computer program can be stored/distributed on any suitable medium, such as an optical storage medium or a solid-state medium supplied together with or as part of other hardware, and can also be distributed in other forms, such as via the Internet or other wired or wireless telecommunication systems. Furthermore, embodiments can take the form of a computer program product accessible from a computer-readable medium providing program code for use by or in connection with a computer or any device or system that executes instructions. For the purposes of this disclosure, a computer-readable medium can generally be any tangible apparatus that can contain, store, communicate, propagate, and/or transport the program for use by or in connection with the instruction execution device. The computer-readable medium can be, for example, without limitation, an electronic, magnetic, optical, electromagnetic, infrared, or semiconductor system, and/or a propagation medium. Non-limiting examples of a computer readable medium include a semiconductor or solid state memory, magnetic tape, a removable computer diskette, a random access memory (RAM), a read-only memory (ROM), a rigid magnetic disk, and/or an optical disk. Optical disks can include compact disk read only memory (CD-ROM), compact disk-read/write (CD-R/W), and/or DVD.

In some embodiments, while shown separately, many of the components of system 100 may be included within the probe 112. In some embodiments, some of the components of system 100 may be included in a tablet or other portable computing device. In some applications, this may provide an ultrasound imaging system that may be more portable and/or more suitable for care in remote/rural environments compared to traditional cart-based ultrasound systems. An example of such a portable system is the Philips^{®} Lumify^{®} system. However, this is merely an example, and the disclosure is not limited to this specific system.

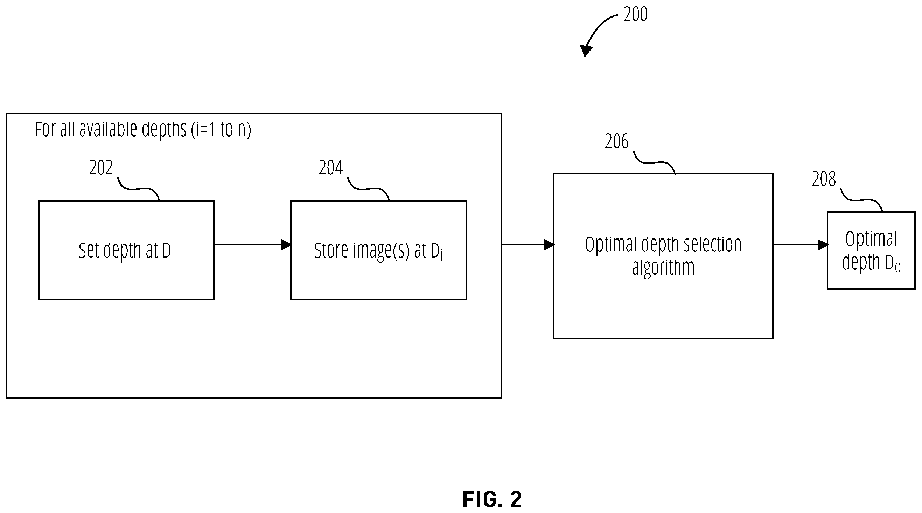

Fig. 2 illustrates an example of a high level optimal depth and zoom estimator 200 for optimal depth and zoom estimation, according to at least one embodiment of the present disclosure.

The optimal depth and zoom estimator 200 may be performed using the system 100 of Fig. 1. For example, the at least one processor 137 may use ultrasound depth images stored in the memory 142 as an input to perform the optimal depth and zoom estimator 200 used for the estimation of an optimal depth setting and/or an optimal zoom setting.

The optimal depth and zoom estimator 200 begins at block 202. At block 202, for all available depths (i= 1 to n) the current depth setting of the ultrasound system may be set at depth Dᵢ. For example, a depth setting of the ultrasound probe 112 (taking ultrasound imaging) may be set at Dᵢ with i incrementing at every iteration of block 202. At block 204, the ultrasound system may store image(s) in the memory 142 acquired at depth Dᵢ. This process may be repeated for all available depths from a first depth D₁, ..., to a final/deepest depth setting Dₙ. Once the images are acquired by the ultrasound probe 112 and stored in the memory 142 at depth Dᵢ, i is incremented by one until n is hit (the final/deepest depth). At block 206, the stored ultrasound image(s) in memory 142 are passed to an optimal depth and zoom estimator performed by the at least one processor 137. At block 208, the optimal depth and zoom estimator performed by the at least one processor 137 outputs an optimal depth setting Dₒ for ultrasound scanning and, in some implementations, an optimal zoom setting. The optimal depth setting and/or the optimal zoom setting may be stored in the memory 142, displayed on the display 138 to the user, or used by the ultrasound probe 112 of the system 100 for future ultrasound scanning.

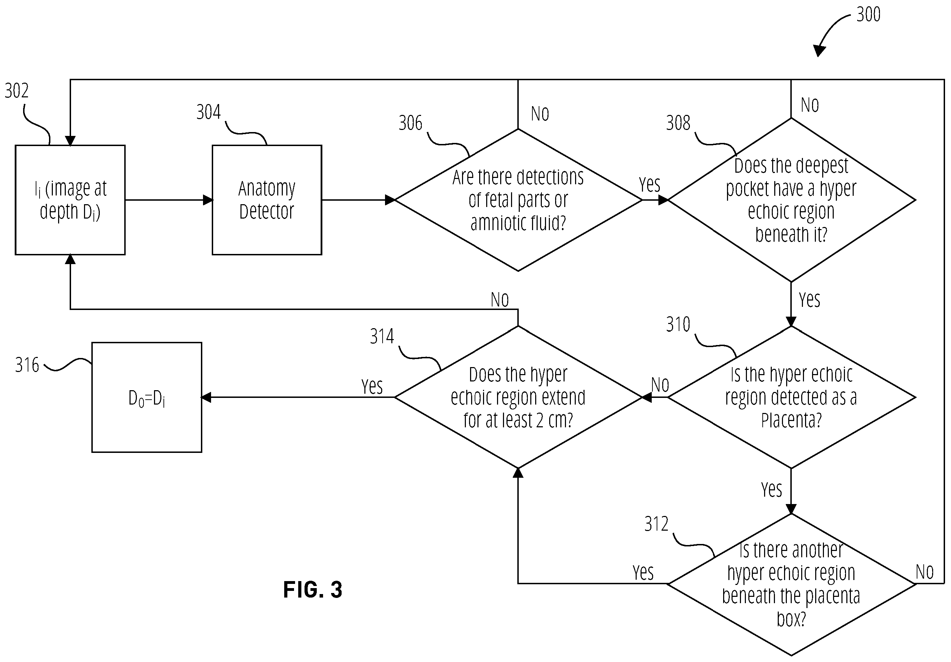

Fig. 3 illustrates an example optimal depth selection AI algorithm 300 , according to at least one embodiment of the present disclosure.

The optimal depth selection AI algorithm 300 may be performed using the system 100 of Fig. 1. For example, the at least one processor 137 may use depth images stored in the memory 142 as an input to perform the optimal depth selection AI algorithm 300 used for the estimation of an optimal depth setting.

The optimal depth selection AI algorithm 300 begins at block 302. At block 302, an image Iᵢ at depth Dᵢ (obtained by the procedure described by the optimal depth and zoom estimator 200 of Fig. 2 and stored in the memory 142) is analyzed. At block 304, the image Iᵢ is analyzed by an anatomy detector using the at least one processor 137 to determine whether an anatomy is detected or not. At block 306, it may be determined whether fetal parts, anatomies or amniotic fluid are detected. At block 306, if no fetal parts, anatomies, and/or amniotic fluid are detected, i is incremented and the next image Iᵢ₊₁ at the depth Dᵢ stored in the memory 142 is to be analyzed at block 302. If the image Iᵢ₊₁ at the depth Dᵢ is the last image acquired/stored at Dᵢ, the depth may be incremented to the next depth Dᵢ₊₁ so that images of the next depth are to be analyzed, until the final/deepest depth Dₙ is analyzed.

At block 306, if fetal parts, anatomies, and/or amniotic fluid are detected, a determination may be made whether the deepest pocket has a hyper echoic region beneath the detected fetal part, anatomy and/or ammonitic fluid, at block 308. At block 308, if it is determined that deepest pocket does not have a hyper echoic region beneath the detected fetal part, anatomy, or amniotic fluid, i is incremented and the next image Iᵢ₊₁ at the depth Dᵢ is to be analyzed at block 302. If image Iᵢ₊₁ at the depth Dᵢ is the last image acquired/stored at Dᵢ, the depth may be incremented to the next depth Dᵢ₊₁ so images of the next depth are to be analyzed. At block 308, if it is determined that deepest pocket does have a hyper echoic region beneath the detected fetal part, anatomy, or amniotic fluid, a determination may be made whether the hyper echoic region detected is a placenta, at block 310.

At block 310, if the hyper echoic region is determined to be a placenta, a determination may be made whether there is another hyper echoic region beneath a bound box of the placenta, at block 312. At block 312, if it is determined that there is no second hyper echoic region beneath the bounding box of the placenta, i is incremented and the next image Iᵢ₊₁ at the depth Dᵢ is to be analyzed at block 302. If image Iᵢ₊₁ at the depth Dᵢ is the last image acquired/stored at Dᵢ, the depth may be incremented to the next depth Dᵢ₊₁ so images of the next depth are to be analyzed. At block 312, it is determined that there is a second hyper echoic region beneath the bounding box of the placenta, a determination may be made if the second hyper echoic region extends for a certain distance (e.g., 2 cm), at block 314.

At block 314, if the second hyper echoic region extends for a certain distance (e.g., 2 cm), the optimal depth setting is Dᵢ and is outputted at block 316. At block 314, if it is determined that the second hyper echoic region does not extend for a certain distance (e.g., 2 cm) i is incremented and the next image Iᵢ₊₁ at the depth Dᵢ is to be analyzed at block 302. If image Iᵢ₊₁ at the depth Dᵢ is the last image acquired/stored at Dᵢ, the depth may be incremented to the next depth Dᵢ₊₁ so images of the next depth are to be analyzed.

At block 310, if the hyper echoic region is determined not to be a placenta, a determination may be made if the detected hyper echoic region that is a placenta extends for a certain distance (e.g., 2 cm), at block 314. At block 314, if the detected hyper echoic region that is a placenta extends for a certain distance (e.g., 2 cm), the optimal depth setting is Dᵢ and is outputted at block 316. At block 314, if it is determined that the detected hyper echoic region that is a placenta does not extend for a certain distance (e.g., 2 cm), i is incremented and the next image Iᵢ₊₁ at the depth Dᵢ is to be analyzed at block 302. If image Iᵢ₊₁ at the depth Dᵢ is the last image acquired/stored at Dᵢ, the depth may be incremented to the next depth Dᵢ₊₁ so that images of the next depth are to be analyzed.

In some implementations, the estimated optimal depth setting may be outputted to the user (e.g., on display 138), stored in memory of the system (e.g., memory 142), or automatically applied for future ultrasound scanning procedures performed by the system 100.

In some ultrasound exams, closer visualizations of certain anatomy may be desired in addition to wider fields of view. For example, while images of the full fetus are desired during a fetal exams, closer visualizations of certain features such as heart, face, abdomen, may be desired. It may be difficult for a novice user to select a proper zoom window within a larger field of view. In some cases, such as during blind sweep protocols, a user may not be capable of selecting a zoom window because the user may not have access to the acquired images. Accordingly, an automatic optimal zoom selection AI algorithm according to principles of the present disclosure may be used.

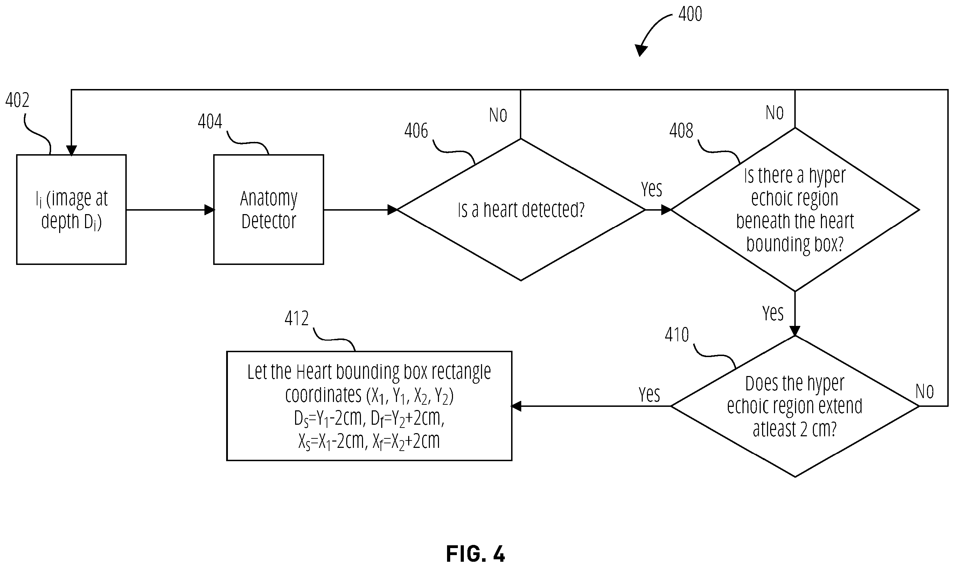

Fig. 4 illustrates an example of an optimal zoom selection AI algorithm 400 used to estimate an optimal zoom setting, according to at least one embodiment of the present disclosure.

The optimal zoom selection AI algorithm 400 may be performed using the system 100 of Fig. 1. For example, the at least one processor 137 may use depth images stored in the memory 142 as an input to perform the optimal zoom selection AI algorithm 400 used for the estimation of an optimal zoom setting. The example in Fig. 4 describes finding a zoom selection for a heart, but other anatomy may be used in other examples.

The optimal zoom selection AI algorithm 400 used to estimate an optimal zoom setting begins at block 402. At block 402, an image Iᵢ at depth Dᵢ stored in memory 142 (which may be obtained by the procedure described by optimal depth and zoom estimator 200) is analyzed. At block 404, the image Iᵢ is analyzed by an anatomy detector using the at least one processor 137 to determine whether an anatomy is detected or not. At block 406, it is determined whether a heart is detected. At block 406, if no, a heart is not detected, i is incremented and the next image Iᵢ₊₁ at the depth Dᵢ is to be analyzed at block 402. If image Iᵢ₊₁ at the depth Dᵢ is the last image acquired/stored at Dᵢ, the depth may be incremented to the next depth Dᵢ₊₁ so images of the next depth are to be analyzed, until the final/deepest depth Dₙ is analyzed.

At block 406, if a heart is detected, a determination may be made whether a hyper echoic region is beneath a bounding box of the heart, at block 408. At block 408, if no hyper echoic region is beneath the bounding box of the heart, i is incremented and the next image Iᵢ₊₁ at the depth Dᵢ is to be analyzed at block 402. If image Iᵢ₊₁ at the depth Dᵢ is the last image acquired/stored at Dᵢ, the depth may be incremented to the next depth Dᵢ₊₁ so that images of the next depth are to be analyzed until all the images including the deepest depth Dₙ are analyzed. At block 408, if the hyper echoic region is beneath the bounding box of the heart, a determination may be made whether the hyper echoic region extends for at least a certain distance (e.g., 2 cm), at block 410. At block 410, if the hyper echoic region does not extend at least a certain distance (e.g., 2 cm), i is incremented and the next image Iᵢ₊₁ at the depth Dᵢ is to be analyzed at block 402. If image Iᵢ₊₁ at the depth Dᵢ is the last image acquired/stored at Dᵢ, the depth may be incremented to the next depth Dᵢ₊₁ so images of the next depth are to be analyzed. At block 410, if the hyper echoic region does extend at least a certain distance (e.g., 2 cm), an optimal zoom setting may be determined based on the certain distance, at block 412. For example, at block 412, if the heart bounding box rectangle coordinates are X₁, Y₁, X₂, Y₂. The optimal zoom setting may be Dₛ=Y₁-2 cm, D_{f}=Y₂+2 cm, Xₛ=X₁-2 cm, and X_{f}=X₂+2 cm where 2 cm is the distance that the hyper echoic region extends over.

In some implementations, the estimated optimal zoom setting may be outputted to the user (e.g., on display 138), stored in memory of the system (e.g., memory 142), or automatically applied for ultrasound scanning procedures performed by the system 100.

It should be understood that the optimal depth selection AI algorithm 300 may be invoked in combination with the optimal zoom selection AI algorithm 400 or independently from the optimal zoom selection AI algorithm 400. Further, the optimal depth setting estimated by the optimal depth selection AI algorithm 300 may be used in combination or independently from the optimal zoom setting estimated by the optimal zoom selection AI algorithm 400.

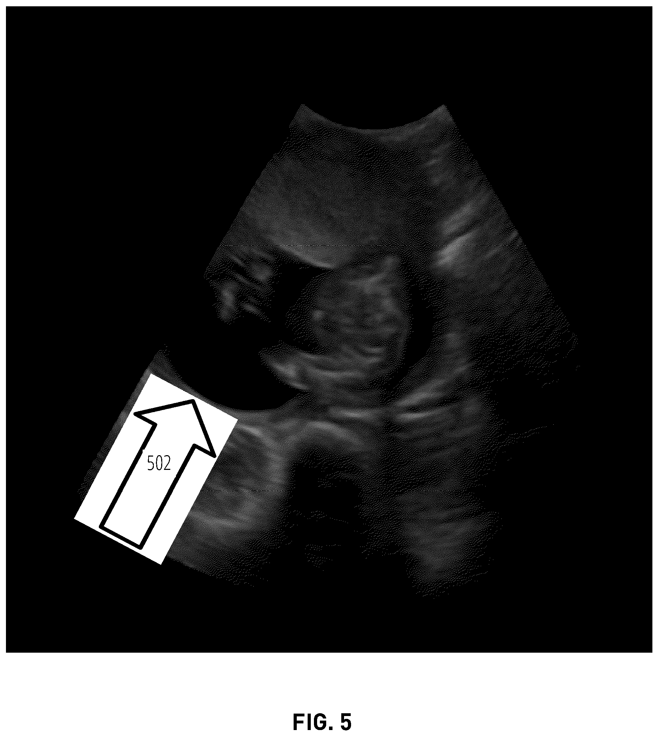

Fig. 5 illustrates an example of a uterine wall as a posterior extent, according to at least one embodiment of the present disclosure.

In some embodiments, a final/deepest depth Dₙ referred to in Fig. 2, Fig. 3 and Fig. 4 may be set and used by the optimal depth selection AI algorithm 300 or the optimal zoom selection AI algorithm 400 in, for example, block 202, block 204, block 302 and block 402 as a posterior extent of the patient. In some examples, the posterior extent may be the posterior uterine wall 502 of the patient.

In some cases, if all images including the final/deepest depth Dₙ are analyzed, it may be assumed that the uterine wall 502 has been detected and no anatomies have been detected between the ultrasound probe and the uterine wall 502. In such cases, the user may be prompted to adjust the position of the probe and perform another ultrasound depth scan.

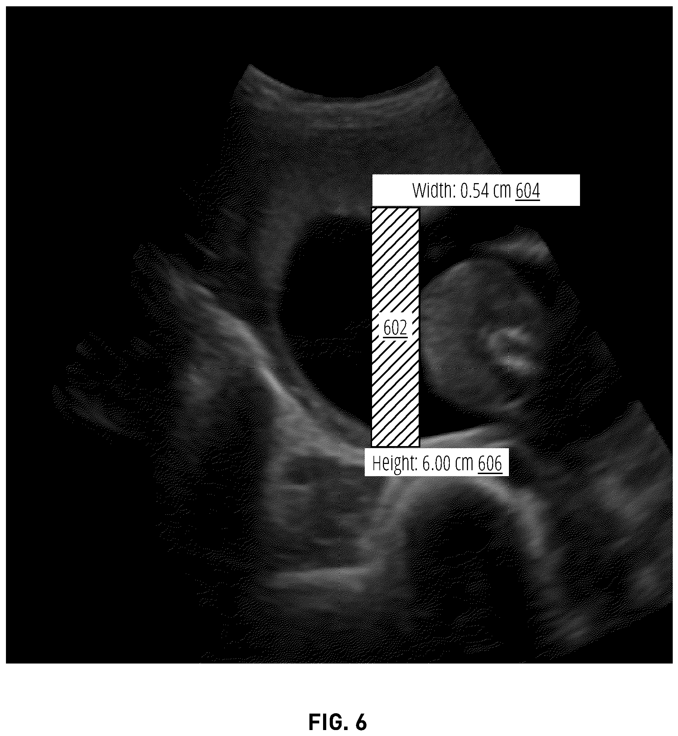

Fig. 6 illustrates an example of a bounding box of amniotic fluid limited by a thin posterior uterine wall, according to at least one embodiment of the present disclosure.

In some examples, the optimal depth selection AI algorithm 300 discussed in Fig. 3 may detect amniotic fluid at, for example, block 306. In some implementations, when amniotic fluid is detected (or any fetal part/anatomy) a bounding box 602 may be applied to the amniotic fluid (or anatomy) and the height 606 and width 604 of the bounding box 602 may be calculated. As an example, the bounding box 602 of the amniotic fluid illustrated in Fig. 6 is calculated to be a width 604 of .54 centimeters (cm) and a height 606 of 6.00 cm.

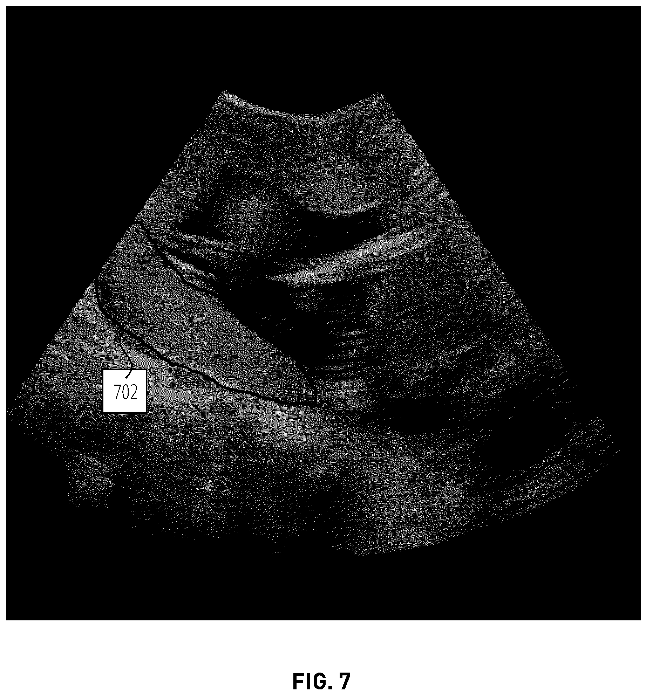

Fig. 7 illustrates an example of a placenta as a posterior anatomy of the uterus, according to at least one embodiment of the present disclosure.

In some embodiments, the optimal depth selection AI algorithm 300 and/or the optimal zoom selection AI algorithm 400 discussed in Fig. 3 and Fig. 4 may determine whether a detected anatomy is a placenta 702 or not in, for example, block 310 or block 406 (where the chosen anatomy is a heart instead of a placenta). Such determination may indicate to the optimal depth selection AI algorithm 300 or the optimal zoom selection AI algorithm 400 that an optimal depth setting or an optimal zoom setting is determined and the optimal depth setting may be used as the depth setting for ultrasound probe 112 of the system 100.

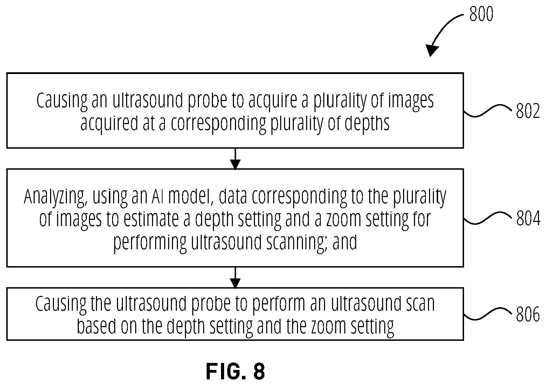

Fig. 8 illustrates a method 800 for determining an ultrasound depth setting and zoom setting, according to at least one embodiment of the present disclosure. The illustrated method 800 includes causing 802 an ultrasound probe to acquire a plurality of images acquired at a corresponding plurality of depths. For example, in block 204 and block 302 the ultrasound probe may acquire the plurality of images.

The method 800 further includes analyzing 804, using an AI model, data corresponding to the plurality of images to estimate a depth setting and a zoom setting for performing ultrasound scanning. For example, in block 206, block 304, block 306, block 308, block 310, block 312, block 314, block 404, block 406, block 408, and block 410 the plurality of images may be analyzed using the AI model.

The method 800 further includes causing 806 the ultrasound probe to perform an ultrasound scan based on the depth setting and the zoom setting. For example, in block 208, block 316, and block 412 an ultrasound scan may be performed based on the depth setting and zoom setting.

In some embodiments, the method 800 further comprises determining whether a fetal anatomy is detected in the ultrasound depth scan at a first depth. For example, in block 304 and block 404 it may be determined whether a fetal anatomy is detected.

In some embodiments, the method 800 further comprises determining whether there is a hyper echoic region beneath the fetal anatomy. For example, in block 308 and block 408, it may be determined whether there is a hyper echoic region beneath the fetal anatomy.

In some embodiments, the method 800 further comprises determining whether the hyper echoic region meets a threshold size. For example, in block 314 and block 410 it may be determined whether the hyper echoic region meets a threshold size.

In some embodiments, the method 800 further comprises determining the zoom setting based on the threshold size. For example, in block 412 a zoom setting is determined.

In some embodiments, the method 800 further comprises, in response to determining that no fetal anatomy is detected at a first depth, determining whether a fetal anatomy is detected in the ultrasound depth scan at a second depth. For example, in block 302, block 304, block 402, and block 404 it may be determined whether a fetal anatomy is detected at a second depth.

The apparatuses, systems and methods disclosed herein may determine an optimal depth and/or an optimal zoom setting for ultrasound scanning. Additionally, the apparatuses, systems and methods disclosed herein may reduce or remove the need for manually adjusting the depth setting and/or zoom setting per individual patient. This automation may reduce the reliance on manual skills and knowledge (or lack thereof), which may allow novice health care workers to perform obstetric ultrasound scanning more efficiently and/or accurately. This may help save time for healthcare professionals and/or improve workflow efficiency. Further, the AI based method to determine optimal depth settings and/or zoom setting for performing ultrasound scans may be integrated into various ultrasound systems or imaging platforms, which may enhance capabilities and/or provide advanced diagnostic tools to novice healthcare providers. It should be understood that an accurate depth setting is important for useful ultrasound image acquisition. Using an incorrect depth setting may hamper the diagnosis of many clinical conditions by not including correct anatomies in question and thus producing non-useful ultrasound imaging.

In various examples where components, systems and/or methods are implemented using a programmable device, such as a computer-based system or programmable logic, it should be appreciated that the above-described systems and methods can be implemented using any of various known or later developed programming languages, such as "Python", "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial, and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices, and methods.

Finally, the above-discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended spirit and scope of the present systems and methods as set forth in the claims that follow. Accordingly, the specification and drawings are to be regarded in an illustrative manner and are not intended to limit the scope of the appended claims.

## Claims

1. A computer-implemented method for determining an ultrasound depth setting and zoom setting, the method comprising:
causing an ultrasound probe to acquire a plurality of ultrasound images at a corresponding plurality of depths in a subject;
analyzing the plurality of ultrasound images to determine a depth setting and a zoom setting for performing an ultrasound scan; and
causing the ultrasound probe to perform the ultrasound scan based on the depth setting and the zoom setting.

2. The computer-implemented method of claim 1, wherein analyzing the plurality of ultrasound images to determine the depth setting and the zoom setting comprises using an artificial intelligence, AI, model to estimate the depth setting and the zoom setting based on data corresponding to the plurality of ultrasound images.

3. The computer-implemented method of claim 1 or 2, further comprising determining whether a fetal anatomy is detected in an ultrasound image of the plurality of ultrasound images at a first depth of the plurality of depths.

4. The computer-implemented method of claim 3, further comprising determining whether there is a first hyper echoic region beneath the fetal anatomy.

5. The computer-implemented method of claim 4, further comprising detecting a deepest pocket in the ultrasound image at the first depth and determining if the deepest pocket comprises the first hyper echoic region.

6. The computer-implemented method of claim 4 or 5, further comprising determining whether the first hyper echoic region meets a threshold size.

7. The computer-implemented method of claim 6, further comprising determining the zoom setting based on the threshold size.

8. The computer-implemented method of any of claims 4 to 7, wherein further comprising determining whether the first hyper echoic region comprises a placenta.

9. The computer-implemented method of any of claims 4 to 8, further comprising determining whether there is a second hyper echoic region beneath the first hyper echoic region.

10. The computer-implemented method of any of claims 3 to 9, further comprising detecting a fetal anatomy based on an anatomy detector model.

11. The computer-implemented method of claim 10, further comprising applying a bounding box to the fetal anatomy.

12. The computer-implemented method of claim 3, further comprising, in response to determining that no fetal anatomy is detected at the first depth, determining whether the fetal anatomy is detected in an ultrasound image of the plurality of ultrasound images at a second depth of the plurality of depths.

13. A non-transitory computer-readable medium carrying instructions that, when executed by at least one processor of an ultrasound imaging system comprising an ultrasound probe configured to acquire ultrasound images, cause the ultrasound imaging system to perform the computer-implemented method of any of claims 1 to 12.

14. An ultrasound imaging system comprising:
an ultrasound probe configured to acquire ultrasound images; and
at least one processor configured to carry out the steps of the method of any of claims 1 to 12.
